# EUROPÄISCHE PATENTSCHRIFT

(11) **EP 4 099 939 B1**
(45) Veröffentlichungstag und Bekanntmachung des Hinweises auf die Patenterteilung: **28.01.2026**
(21) Anmeldenummer: 21704460.1
(22) Anmeldetag: 05.02.2021
(51) Int. Cl.: A61B 50/30, A61B 50/33, A61B 50/34, A61B 90/90, A61B 90/92, A61B 50/00

(54) **KENNZEICHNUNGSSYSTEM FÜR STERILCONTAINER UND SIEBSCHALEN**
IDENTIFICATION SYSTEM FOR STERILE CONTAINERS AND MESH TRAYS
SYSTÈME D'IDENTIFICATION POUR RÉCIPIENTS STÉRILES ET PLATEAUX À MAILLES

(30) Priorität: 07.02.2020 DE 102020103131
(43) Veröffentlichungstag der Anmeldung: 14.12.2022
(73) Patentinhaber: Aesculap AG, 78532 Tuttlingen (DE)
(72) Erfinder: ZIERIS, Gerold, 78532 Tuttlingen - Möhringen (DE); BOHNENSTENGEL, Philipp, 78256 Steißlingen (DE); HENKE, Matthias, 78048 Villingen-Schwenningen (DE); FRECH, Bozica, 78598 Königsheim (DE); MALIGLOWKA, Johann, 78600 Kolbingen (DE); STEINER, Sabrina, 78652 Deisslingen (DE); BAUER, Stephan, 78576 Emmingen (DE); MOTZ, Corvin, 88630 Pfullendorf (DE); AMANN, Joachim, 78357 Mühlingen - Zoznegg (DE); SCHEIT, Michael, 72770 Reutlingen (DE); HÖFLER, Martina, 78647 Trossingen (DE)
(74) Vertreter: Winter, Brandl - Partnerschaft mbB
(86) Internationale Anmeldenummer: PCT/EP2021/052740
(87) Internationale Veröffentlichungsnummer: WO 2021/156405

(56) Entgegenhaltungen:
- WO-A1-2018/104390
- WO-A1-2019/117928
- WO-A1-2020/011933

## Beschreibung

Die vorliegende Erfindung betrifft ein Kennzeichnungssystem für medizintechnische Sterilcontainer und Siebschalen zur Aufnahme medizintechnischer Instrumente, umfassend eine Mehrzahl an Sterilcontainern, eine Mehrzahl an Siebschalen und eine Mehrzahl an Kennzeichnungsschildern.

Im Rahmen eines Instrumentenkreislaufs einer medizinischen Einrichtung wie einem Krankenhaus, einer Klinik oder einer ZSVA (zentrale Sterilgutversorgungsabteilung) ist es an verschiedenen Stellen erforderlich, sowohl Sterilcontainer als auch Sterilisiersiebschalen / Siebschalen gut und flexibel zu kennzeichnen. Zu diesem Zweck ist eine Reihe von Kennzeichnungssystemen bekannt, von denen allerdings keine einen ganzheitlichen Ansatz verfolgt. Vielmehr gibt es eine Vielzahl unterschiedlicher Kennzeichnungssysteme, die eine medizinische Einrichtung in nachteiliger Weise vorhalten und anwenden muss.

Ein solches bekanntes Kennzeichnungssystem weist zum Beispiel Sterilcontainer mit in der Regel fest verbauten Halterungen für Kennzeichnungsschilder auf. In diese Halterungen können die jeweils erforderlichen Kennzeichnungsschilder hineingeschoben werden, um den im Sterilcontainer befindlichen Inhalt zu identifizieren.

Es sind außerdem Sonderkennzeichnungen zur außerordentlichen Kennzeichnung von Sterilcontainern und Siebschalen bekannt. Für einen solchen Zweck werden in der Regel Anhänger oder Etiketten verwendet, die mit entsprechenden Vermerken wie zum Beispiel einer Aufschrift "infektiös", "Achtung unvollständig", etc. versehen sind, und die flexibel am jeweiligen Sterilcontainer oder an der jeweiligen Siebschale angebracht und entfernt werden können.

Zur Kennzeichnung von sterilisierten Siebschalen und Identifikation des darin enthaltenen Inhalts sind insbesondere Kennzeichnungsschilder bekannt, die in unterschiedlicher Weise flexibel an der Siebschale zu befestigen sind. Zum Beispiel können solche Kennzeichnungsschilder auf den Rand der Siebschale aufsteckbar sein, mit der Siebschale verschraubt werden oder die Netzstruktur der Siebschale durch- und hintergreifende Drahtbefestigungen aufweisen. Beispielsweise zeigt WO 2019 / 117 928 A1 eine austauschbare Kennzeichnungsplatte zur lösbaren Befestigung an einem Sterilcontainer. Weiterer Stand der Technik ist aus der WO 2018 / 104 390 A1 sowie der WO 2020 / 011 933 A1 bekannt.

Es sind außerdem Systeme für eine Kennzeichnung von noch aufzubereitenden Instrumenten bekannt. In der Regel wird das Instrumentarium nach einer OP für eine Aufbereitung sortiert und auf mehrere Siebschalen aufgeteilt. Um zu erkennen, welche Siebschalen zueinander gehören, werden diese mit sogenannten Entsorgungsmarken ausgestattet. Diese sind häufig als elastische Klammern ausgebildet, die einfach auf den Siebschalenrand angesteckt werden können. Eine andere Möglichkeit besteht in einer Verwendung einfacher Chips oder Marken, die einfach in die jeweiligen Siebschalen hineingelegt werden.

Bei dem vorstehend beschriebenen Stand der Technik ist von Nachteil, dass es kein einheitliches Kennzeichnungssystem für Sterilcontainer und Siebschalen gibt, mit dem alle Anwendungsfälle abgedeckt werden können. Das einzige System, das sowohl an Sterilcontainern als auch an Siebschalen verwendet und flexibel angebracht und entfernt werden kann, sind Anhänger. Diese sind allerdings nicht zur Aufbereitung geeignet und eher störend, da sie in der Regel im Griffbereich angebracht werden.

Vor diesem Hintergrund liegt der vorliegenden Erfindung die Aufgabe zugrunde, die genannten Nachteile des Stands der Technik zu verringern, insbesondere ein ganzheitliches System zur flexiblen Kennzeichnung sowohl von Sterilcontainern als auch von Sterilisiersiebschalen in einer ZSVA- und/oder Klinik-Umgebung zu schaffen.

Diese Aufgabe wird nach der vorliegenden Erfindung gelöst durch ein Kennzeichnungssystem nach Anspruch 1, also ein Kennzeichnungssystem für medizintechnische Sterilcontainer und Siebschalen zur Aufnahme medizintechnischer Instrumente, umfassend eine Anzahlzahl (mindestens ein oder mehrere) an Sterilcontainern, eine Anzahl (mindestens ein oder mehrere) an Siebschalen und eine Anzahl (mindestens ein oder mehrere) an Kennzeichnungsschildern, wobei das/jedes Kennzeichnungsschild eine Schnittstelle aufweist, an dem/jedem Sterilcontainer und durch jede Siebschale zumindest eine zur Schnittstelle komplementäre Gegenschnittstelle ausgebildet ist, und die Schildschnittstelle und die Gegenschnittstelle zum haltenden Anordnen zumindest eines Kennzeichnungsschilds an dem/einem der Sterilcontainer bzw. an der/einem der Siebschalen ausgebildet sind. Insbesondere umfassen die Schnittstelle und die Gegenschnittstelle miteinander in Eingriff bringbare komplementäre Raststrukturen. Diese Raststrukturen können insbesondere lösbar miteinander in Eingriff bringbar sein. Dabei ist die Schnittstelle erfindungsgemäß in Form zumindest eines Rastvorsprungs ausgebildet. Die Gegenschnittstelle ist dann in Form zumindest einer Rastausnehmung ausgebildet. Derartige Raststrukturen sind einfach und kostengünstig herzustellen und bieten insbesondere eine robuste und anwenderfreundliche Möglichkeit, ein Kennzeichnungsschild an einem Sterilcontainer, einer Siebschale oder einem Adapterelement anzuordnen und davon wieder zu lösen / entfernen. An zumindest einem Sterilcontainer der Mehrzahl an Sterilcontainern ist ein Adapterelement angeordnet. Vorzugsweise ist das Adapterelement lösbar an dem Sterilcontainer angeordnet. Das Adapterelement bildet die Gegenschnittstelle aus. Alternativ kann die Gegenschnittstelle durch das Adapterelement ausgebildet sein. Mittels eines solchen Adapterelements kann die Erfindung in vorteilhafter Weise mit üblichen Sterilcontainern genutzt werden. Zum Beispiel kann das Adapterelement an bestehenden Behälterverschlüssen oder Kennzeichnungsträgern von bekannten Sterilcontainern angeordnet werden.

Vorteilhafte Ausführungsformen der Erfindung sind in den Unteransprüchen beansprucht und werden ebenfalls nachfolgend näher erläutert.

Die Erfindung ermöglicht in vorteilhafter Weise ein einheitliches / ganzheitliches Kennzeichnungssystem, mit dem Kennzeichnungsschilder besonders einfach austauschbar sowohl an einem Sterilcontainer wie auch an einer Siebschale angebracht werden können, so dass eine systemumfassende Kennzeichnung von aufbereiteten / sterilisierten wie auch aufzubereitenden / zu sterilisierenden medizinischen Instrumenten problemlos möglich ist. Die Erfindung schafft zu diesem Zweck einen ganzheitlichen Ansatz, so dass eine medizinische Einrichtung wie eine Klinik, ein Krankenhause und/oder eine ZSVA lediglich noch das erfindungsgemäße Kennzeichnungssystem vorhalten und anwenden muss, um Instrumente in deren gesamten Nutzungszyklus dauerhaft und verlässlich zu kennzeichnen. Die Kennzeichnung erfolgt im Rahmen der Erfindung dabei in einer solchen Weise, dass Aufbereitungs-, Reinigungs- und/oder Sterilisierungsvorgänge und - abläufe nicht behindert werden. Ein weiterer Vorteil ist, dass nicht nur die jeweiligen Instrumente gekennzeichnet werden können, sondern auch die Zugehörigkeit von Sterilcontainern und Siebschalen zueinander.

Die Kennzeichnungsschilder können im Rahmen der Erfindung als übliche Codierungen ausgebildet sein. Insbesondere kann das erfindungsgemäße System Kennzeichnungsschilder von jeweils unterschiedlicher Farbe aufweisen. Die Kennzeichnungsschilder können alternativ oder zusätzlich mit Beschriftung und/oder maschinenlesbaren Codes versehen sein. Da sie besonders einfach anzuordnen und zu entfernen sind, können sie außerdem als Sonderkennzeichnungen mit entsprechenden Vermerken oder beschriftbar ausgebildet sein. Vorzugsweise bestehen die Kennzeichnungsschilder aus Kunststoff oder Metall.

Eine Ausführungsform des erfindungsgemäßen Kennzeichnungssystems ist als Codesystem ausgebildet, indem die Anzahl (vorzugsweise Mehrzahl) an Kennzeichnungsschildern Gruppen von Kennzeichnungsschildern von jeweils unterschiedlicher Farbe aufweist. Auf diese Weise lässt sich die Erfindung einfach im Rahmen von bestehenden Instrumentenorganisationsstrukturen verwenden.

Es ist von besonderem Vorteil, wenn an zumindest einem Sterilcontainer und/oder durch einer Siebschale jeweils eine Anzahl (vorzugsweise Mehrzahl) von Gegenschnittstellen ausgebildet ist. Auf diese Weise können mehrere Kennzeichnungsschilder an einem Sterilcontainer und/oder einer Siebschale angeordnet werden, so dass eine Vielzahl von Informationen und/oder Codierungen in einfacher Weise übertragen werden können. Die Gegenschnittstellen können insbesondere derart ausgebildet und/oder ausgerichtet sein, dass ein Halten von Kennzeichnungsschildern in unterschiedlichen Positionen möglich ist.

An zumindest einem Kennzeichnungsschild können als Schnittstelle zwei voneinander beabstandete hakenförmige Rastnasen ausgebildet sein. Diese weisen vorzugsweise jeweils eine an dem Kennzeichnungsschild angeordnete Rastnasenbasis und einen sich von dieser an deren von dem Kennzeichnungsschild abgewandten Seite erstreckenden Rasthaken auf. Die Rasthaken sind vorzugsweise einander zugewandt oder voneinander abgewandt. Zwischen den Rastnasen ist vorzugsweise ein elastisch verformbarer Abschnitt ausgebildet. Ein derartiges Kennzeichnungsschild ist besonders einfach und anwenderfreundlich an einem Sterilcontainer bzw. an einer Siebschale anzuordnen und davon zu lösen, indem zumindest eine der Rastnasen durch Verformen des elastisch verformbaren Abschnitts gegenüber der entsprechenden Rastausnehmung außer Eingriff gebracht werden kann.

Es ist dazu von besonderem Vorteil, wenn das Kennzeichnungsschild zumindest einen Griffabschnitt aufweist, an dem es nutzerseitig zu ergreifen und durch eine entsprechende Kraft elastisch verformbar ist. Der Griffabschnitt kann insbesondere randseitig auf der dem verformbaren Abschnitt gegenüberliegenden Seite der entsprechenden Rastnase angeordnet sein. Derart kann das Kennzeichnungsschild besonders einfach ergriffen und verformt werden.

Eine weitere Ausführungsform der Erfindung ist dadurch gekennzeichnet, dass die Gegenschnittstelle zumindest einer Siebschale in Form von zumindest einer in eine Siebschalenwandung eingebrachte Rastausnehmung ausgebildet ist.

Das Kennzeichnungssystem nach der Erfindung kann außerdem zumindest eine Transporteinrichtung und/oder zumindest eine Lagereinrichtung und/oder zumindest ein Reinigungs- und Desinfektionsrack umfassen. Diese sind jeweils mit einer zur Schnittstelle komplementären Gegenschnittstelle versehen. Auf diese Weise kann die Handhabung von sterilisierten / aufbereiteten oder zu sterilisierenden / aufzubereitenden medizinischen Instrumenten sowie ein mit der Erfindung versehenes klinisches Aufbereitungssystem umfassen vereinfacht werden.

Man kann sagen, dass die Erfindung ein einheitliches Kennzeichnungssystem sowohl für Sterilcontainer als auch für Sterilisiersiebschalen / Siebschalen zur Verfügung stellt, das insbesondere flexibel angebracht und entfernt werden kann. Das erfindungsgemäße System kann insbesondere verschiedenfarbige Kennzeichnungsschilder mit Koppelschnittstellen zum Beispiel in Form von Rastnasen und eine entsprechende Gegenschnittstelle aufweisen, die einfach an unterschiedlichen Produkten wie zum Bespiel einer Frontblende eines Sterilcontainers, einer Sterilisiersiebschale, etc., umgesetzt werden kann. Die Koppelschnittstellen und die Gegenschnittstellen können insbesondere eine Art Klick-System ausbilden, mit dem die Kennzeichnungsschilder besonders einfach und nutzerfreundlich flexibel angebracht und entfernt werden können. Durch das vereinheitlichte Kennzeichnungssystem können alle genannten Kennzeichnungsszenarien (siehe Kapitel "Problembeschreibung") abgedeckt werden.

Die Erfindung ist ein einheitliches Kennzeichnungssystem für Sterilcontainer und Sterilisiersiebschalen bestehend aus verschiedenfarbigen Kennzeichnungsschildern, die mit Rastnasen ausgestattet sind, und einer entsprechenden Schnittstelle, die an unterschiedlichen Produkten (z.B. Frontblende von Sterilcontainer, Sterilisiersiebschale, etc.) zum Einsatz kommt. Es sei darauf hingewiesen, dass das Kennzeichnungssystem außerdem einen Sterilcontainerdeckel aufweisen kann und dass das Kennzeichnungsschild und/oder das Adapterelement an diesem angeordnet sein kann.

Weitere Merkmale und Vorteile der vorliegenden Erfindung ergeben sich aus der folgenden beispielhaften und nicht beschränkenden Beschreibung der Figuren. Diese sind lediglich schematischer Natur und dienen nur dem Verständnis der Erfindung. Dabei zeigen:
Fig. 1 eine Sterilcontainer-Siebschalen-Einheit mit einem Kennzeichnungssystem nach der Erfindung in einer schematischen perspektivischen Ansicht,
Fig. 2 eine Ausführungsform eines Kennzeichnungsschilds eines Kennzeichnungssystems nach der Erfindung in einer schematischen perspektivischen Ansicht,
Fig. 3 eine Ausführungsform eines im Rahmen der Erfindung an einem Sterilcontainer angeordnetem Adapterelement in einer perspektivischen Ansicht, und
Fig. 4 einen Ausschnitt einer Siebschale eines Kennzeichnungssystems nach der Erfindung.

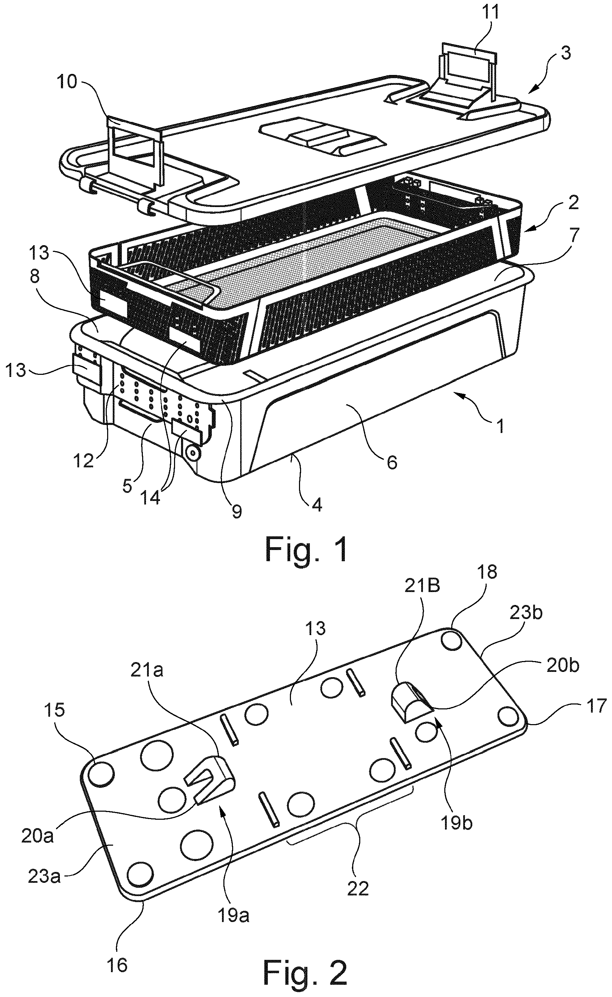

Figur 1 zeigt eine Sterilcontainer-Siebschalen-Einheit mit einem Sterilcontainer 1, einer Siebschale 2 und einem Sterilcontainerdeckel 3. Die Sterilcontainer-Siebschalen-Einheit ist Teil eines Kennzeichnungssystems nach der Erfindung.

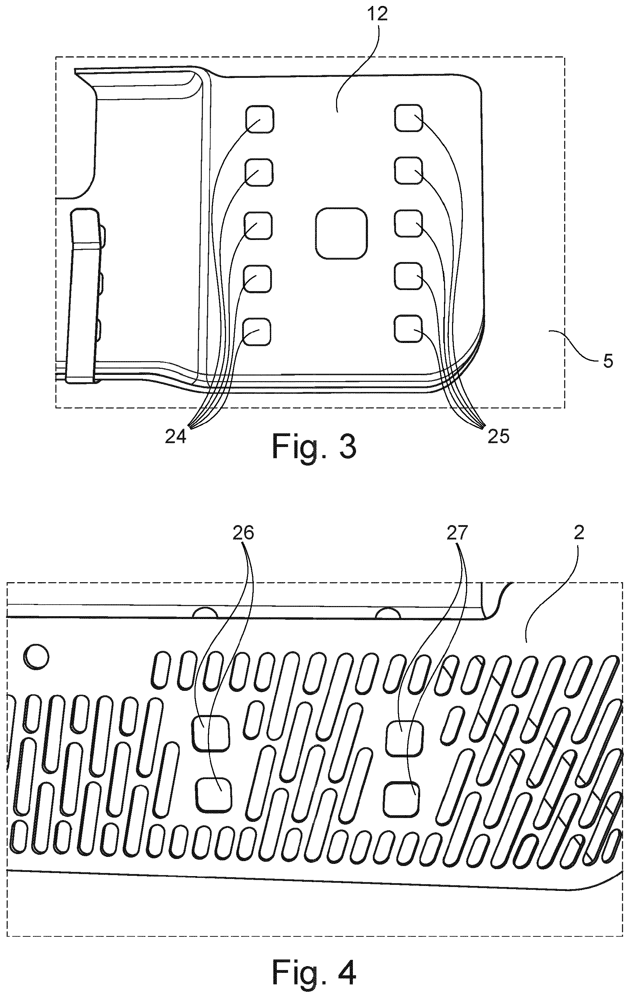

Der Sterilcontainer 1 weist einen Containerboden 4 und daran angeordnete Containerwände 5, 6, 7, 8 auf und bildet ein Aufnahmevolumen für die Siebschale 2 aus. Die Containerwände 5, 6, 7, 8 bilden zusammen einen Containerrand 9, auf den der Containerdeckel 3 dicht anzuordnen ist. Der Containerdeckel 3 ist mit Verschlüssen 10, 11 in an sich bekannter Weise mit dem Sterilcontainer 1 verriegelbar. An der Containerwand 5 ist ein Adapterelement 12 angeordnet (siehe auf Figur 3). Das Kennzeichnungssystem weist außerdem eine Mehrzahl von Kennzeichnungsschildern 13, 14 jeweils in Form von farbigen Kunststoffspritzgussteilen auf.

Eines der Kennzeichnungsschilder 13 ist in Figur 2 perspektivisch einzeln dargestellt. Dieses ist im Wesentlichen rechteckig mit abgerundeten Ecken 15, 16, 17, 18 ausgebildet. An seiner in Figur 2 gezeigten Unterseite (also der bei bestimmungsgemäßer Anordnung zum Sterilcontainer bzw. zur Siebschale weisenden Seite) sind eine erste Raststruktur 19a und eine zweite Raststruktur 19b als voneinander beabstandete hakenförmige Rastnasen 19a, 19b angeordnet, hier angespritzt. Jede der Raststrukturen 19a, 19b weist eine an dem Kennzeichnungsschild 13 angeordnete Rastnasenbasis 20a, 20b und eine sich von dieser an deren von dem Kennzeichnungsschild 13 abgewandten Seite erstreckenden Rasthaken 21a, 21b auf. Die Rasthaken 21a, 21b sind einander zugewandt angeordnet. Zwischen den Rastnasen 19a, 19b weist das Kennzeichnungsschild 13 einen elastisch verformbaren Abschnitt 22 auf. Die Kurzseiten des Kennzeichnungsschilds 13 zwischen den Ecken 15 und 16 bzw. 17 und 18 sind als Griffelemente 23a, 23b ausgebildet, indem das das Material des Kennzeichnungsschilds 13 aus der Ebene des Abschnitts 22 herausgebogen ist. Die beiden Raststrukturen 19a, 19b bilden die Schnittstellen des Kennzeichnungsschilds 13 im Sinne der Erfindung aus.

In Figur 3 ist ein Ausschnitt des Adapterelements 12 gezeigt. Dieses weist eine Mehrzahl an in Form von Durchgangsöffnungen 24, 25 ausgebildeten Rastöffnungen 24, 25 auf. Diese bilden Gegenschnittstellen im Sinne der Erfindung. Die Rastöffnungen 24 sind komplementär zu den Rastnasen 13, während die Rastöffnungen 25 komplementär zu den Rastnasen 14 sind.

Figur 4 zeigt einen Ausschnitt der Siebschale 2. Auch diese weist eine Mehrzahl an in Form von Durchgangsöffnungen 26, 27 ausgebildeten Rastöffnungen 26, 27 auf. Diese bilden Gegenschnittstellen im Sinne der Erfindung. Die Rastöffnungen 26 sind komplementär zu den Rastnasen 13, während die Rastöffnungen 27 komplementär zu den Rastnasen 14 sind.

Wie insbesondere Figur 1 zu entnehmen ist, können die Kennzeichnungsschilder 13, 14 sowohl in den Rastöffnungen / Gegenschnittstellen 26 bzw. 27 der Siebschale 2 als auch in den Rastöffnungen / Gegenschnittstellen 24, 25 des Sterilcontainers 1 platziert und angeordnet werden. Dabei gelangen die Rastnasen 19a, 19b mit den entsprechenden Rastöffnungen / Gegenschnittstellen 24, 25, 26, 27 in Eingriff, wobei die Rasthaken 21a, 21b durch die Rastöffnungen / Gegenschnittstellen 24, 25, 26, 27 hindurchgeführt sind und diese hintergreifen. Beim Hindurchführen der Rasthaken 21a, 21b kommt es zu einer elastischen Verformung des Abschnitts 22 des jeweiligen Kennzeichnungsschilds 13, 14. Sind die Rasthaken 21a, 21b durch die entsprechenden Rastöffnungen / Gegenschnittstellen 24, 25, 26, 27 hindurchgeführt, federt der Abschnitt 22 infolge seiner elastischen Eigenschaften zurück in seine ursprüngliche Form, so dass die Rasthaken 21a, 21b die Rastöffnungen / Gegenschnittstellen 24, 25, 26, 27 hintergreifen und das Kennzeichnungsschild 13, 14 an dem Sterilcontainer 1 bzw. der Siebschale 2 halten. Ein Lösen / Entfernen des Kennzeichnungsschilds 13, 14 erfolgt in entsprechender Weise umgekehrt.

Die vorliegende Erfindung betrifft zusammenfassend ein Kennzeichnungssystem für medizintechnische Sterilcontainer 1 und Siebschalen 2 zur Aufnahme medizintechnischer Instrumente, umfassend eine Anzahl (d.h. ein oder mehrere) an Sterilcontainern 1, eine Anzahl (d.h. ein oder mehrere) an Siebschalen 2 und eine Anzahl (d.h. ein oder mehrere an Kennzeichnungsschildern 13, 14, wobei jedes Kennzeichnungsschild 13, 14 eine Schnittstelle 19a, 19b aufweist, an jedem Sterilcontainer 1 und jeder Siebschale 2 zumindest eine zur Schnittstelle 19a, 19b komplementäre Gegenschnittstelle 24, 25, 26, 27 ausgebildet oder angeordnet ist, und die Schnittstelle 19a, 19b und die Gegenschnittstelle 24, 25, 26, 27 zum haltenden Anordnen zumindest eines Kennzeichnungsschilds 13, 14 an einem der Sterilcontainer 1 bzw. an einer der Siebschalen 2 ausgebildet sind.

### Bezugszeichenliste

- 1: Sterilcontainer
- 2: Siebschale
- 3: Sterilcontainerdeckel
- 4: Containerboden
- 5: Containerwand
- 6: Containerwand
- 7: Containerwand
- 8: Containerwand
- 9: Containerrand
- 10: Verschluss
- 11: Verschluss
- 12: Adapterelement
- 13: Kennzeichnungsschild
- 14: Kennzeichnungsschild
- 15: Ecke
- 16: Ecke
- 17: Ecke
- 18: Ecke
- 19a, b: Raststruktur, Rastnase
- 20a, b: Rastnasenbasis
- 21a, b: Rasthaken
- 22: elastisch verformbarer Abschnitt
- 23a, b: Griffelement
- 24: Durchgangsöffnung, Rastöffnung
- 25: Durchgangsöffnung, Rastöffnung
- 26: Durchgangsöffnung, Rastöffnung
- 27: Durchgangsöffnung, Rastöffnung

## Patentansprüche

1. Kennzeichnungssystem für medizintechnische Sterilcontainer (1) und Siebschalen (2) zur Aufnahme medizintechnischer Instrumente, umfassend eine Anzahl an Sterilcontainern (1), eine Anzahl an Siebschalen (2) und eine Anzahl an Kennzeichnungsschildern (13, 14),
wobei
jedes Kennzeichnungsschild (13, 14) eine Schnittstelle (19a, 19b) aufweist,
an jedem Sterilcontainer (1) und durch jede Siebschale (2) zumindest eine zur Schnittstelle (19a, 19b) komplementäre Gegenschnittstelle (24, 25, 26, 27) ausgebildet ist, und
die Schnittstelle (19a, 19b) und die Gegenschnittstelle (24, 25, 26, 27) zum haltenden Anordnen zumindest eines Kennzeichnungsschilds (13, 14) an einem der Sterilcontainer (1) und an einer der Siebschalen (2) ausgebildet sind,
wobei die Schnittstelle (19a, 19b) in Form von zumindest einem Rastvorsprung (19a, 19b) und die Gegenschnittstelle (24, 25, 26, 27) in Form zumindest einer Rastausnehmung (24, 25, 26, 27) ausgebildet ist,
an zumindest einem Sterilcontainer (1) der Mehrzahl an Sterilcontainern (1) ein Adapterelement (12) angeordnet ist, insbesondere lösbar angeordnet ist, welches die Gegenschnittstelle (24, 25) ausbildet oder durch das die Gegenschnittstelle (24, 25) ausgebildet ist, und
an zumindest einem Sterilcontainer (1) und/oder durch zumindest eine Siebschale (2) jeweils eine Anzahl von Gegenschnittstellen (24, 25, 26, 27) ausgebildet ist, die ein Halten von Kennzeichnungsschildern (13, 14) in unterschiedlichen Positionen ermöglichen.

2. Kennzeichnungssystem nach Anspruch 1, **dadurch gekennzeichnet, dass** die Kennzeichnungsschilder (13, 14) aus Kunststoff bestehen.

3. Kennzeichnungssystem nach Anspruch 1 oder 2, **dadurch gekennzeichnet, dass** die Anzahl an Kennzeichnungsschildern (13, 14) Gruppen von Kennzeichnungsschildern (13, 14) von jeweils unterschiedlicher Farbe aufweist.

4. Kennzeichnungssystem nach einem der vorstehenden Ansprüche, **dadurch gekennzeichnet, dass** die Schnittstelle (19a, 19b) und die Gegenschnittstelle (24, 25, 26, 27) miteinander in Eingriff bringbare, insbesondere lösbar miteinander in Eingriff bringbare, komplementäre Raststrukuren umfassen.

5. Kennzeichnungssystem nach einem der vorstehenden Ansprüche, **dadurch gekennzeichnet, dass** an zumindest einem Kennzeichnungsschild (13, 14) als Schnittstelle (19a, 19b) zwei voneinander beabstandete hakenförmige Rastnasen (19a, 19b) ausgebildet sind, die jeweils eine an dem Kennzeichnungsschild (13, 14) angeordnete Rastnasenbasis (20a, 20b) und eine sich von dieser an deren von dem Kennzeichnungsschild (13, 14) abgewandten Seite erstreckenden Rasthaken (21a, 21b) aufweisen, wobei die Rasthaken (21a, 21b) einander zugewandt oder voneinander abgewandt sind und zwischen den Rastnasen (19a, 19b) ein elastisch verformbarer Abschnitt (22) ausgebildet ist.

6. Kennzeichnungssystem nach dem vorstehenden Anspruch, **dadurch gekennzeichnet, dass** das Kennzeichnungsschild (13, 14) einen Griffabschnitt (23a, 23b) aufweist, der insbesondere randseitige auf der dem verformbaren Abschnitt (22) gegenüberliegenden Seite der entsprechenden Rastnase (19a, 19b) angeordnet ist.

7. Kennzeichnungssystem nach einem der vorstehenden Ansprüche, **dadurch gekennzeichnet, dass** die Gegenschnittstelle (26, 27) zumindest einer Siebschale (2) in Form von zumindest einer in eine Siebschalenwandung eingebrachte Rastausnehmung (26, 27) ausgebildet ist.

8. Kennzeichnungssystem nach einem der vorstehenden Ansprüche, außerdem umfassend zumindest eine Transporteinrichtung und/oder zumindest eine Lagereinrichtung und/oder zumindest ein Reinigungs- und Desinfektionsrack, jeweils mit einer zur Schnittstelle (19a, 19b) komplementären Gegenschnittstelle (24, 25, 26, 27).

## Claims

1. An identification system for medical sterile containers (1) and sieve trays (2) for holding medical instruments, comprising a number of sterile containers (1), a number of sieve trays (2) and a number of identification plates (13, 14),
wherein
each identification plate (13, 14) has an interface (19a, 19b),
at least one counterpart interface (24, 25, 26, 27) complementary to the interface (19a, 19b) is formed on each sterile container (1) and by each sieve tray (2), and
the interface (19a, 19b) and the counterpart interface (24, 25, 26, 27) are formed for holding at least one identification plate (13, 14) on one of the sterile containers (1) and on one of the sieve trays (2), respectively,
wherein
the interface (19a, 19b) is configured in the form of at least one latching projection (19a, 19b) and the counterpart interface (24, 25, 26, 27) is configured in the form of at least one latching recess (24, 25, 26, 27),
an adapter element (12) is arranged, in particular detachably arranged, on at least one sterile container (1) of the plurality of sterile containers (1), wherein the adapter element (12) forms the counterpart interface (24, 25) or is formed by the counterpart interface (24, 25), and
a number of counterpart interfaces (24, 25, 26, 27) is formed on at least one sterile container (1) and/or by at least one sieve tray (2), which makes it possible to hold identification plates (13, 14) in different positions.

2. The identification system according to claim 1, **characterized in that** the identification plates (13, 14) are made of plastic.

3. The identification system according to claim 1 or 2, **characterized in that** the number of identification plates (13, 14) comprises groups of identification plates (13, 14) each of a different color.

4. The identification system according to one of the preceding claims, **characterized in that** the interface (19a, 19b) and the counterpart interface (24, 25, 26, 27) comprise complementary latch structures that are engageable with each other, in particular releasably engageable with each other.

5. The identification system according to one of the preceding claims, **characterized in that** two spaced-apart, hook-shaped snap-in noses (19a, 19b) are formed as interface (19a, 19b) on at least one identification plate (13, 14), wherein these snap-in noses each have a snap-in nose base (20a, 20b) arranged on the identification plate (13, 14) and a latch hook (21a, 21b) extending from this base on its side facing away from the identification plate (13, 14), wherein the latch hooks (21a, 21b) face towards or away from each other and an elastically deformable portion (22) is formed between the snap-in noses (19a, 19b).

6. The identification system according to the preceding claim, **characterized in that** the identification plate (13, 14) comprises a grip portion (23a, 23b) arranged in particular at the edge on the side of the corresponding snap-in nose (19a, 19b) opposite to the deformable portion (22).

7. The identification system according to one of the preceding claims, **characterized in that** the counterpart interface (26, 27) of at least one sieve tray (2) is in the form of at least one detent recess (26, 27) formed in a sieve tray wall.

8. The identification system according to one of the preceding claims, further comprising at least one transport device and/or at least one storage device and/or at least one cleaning and disinfection rack, each with a counterpart interface (24, 25, 26, 27) complementary to the interface (19a, 19b).

## Revendications

1. Système de marquage pour récipients stériles médicaux (1) et tamis (2) destinés à accueillir des instruments médicaux, comprenant une pluralité de récipients stériles (1), une pluralité de tamis (2) et une pluralité de plaques de marquage (13, 14),
dans lequel
chaque plaque de marquage (13, 14) présente une interface (19a, 19b), au moins une contre-interface (24, 25, 26, 27) complémentaire de l'interface (19a, 19b) est réalisée au niveau de chaque récipient stérile (1) et à travers chaque tamis (2), et
l'interface (19a, 19b) et la contre-interface (24, 25, 26, 27) sont réalisées pour
maintenir en place au moins une plaque de marquage (13, 14) au niveau de l'un des récipients stériles (1) et au niveau de l'un des tamis (2),
dans lequel
l'interface (19a, 19b) est réalisée sous la forme d'au moins une saillie d'encliquetage (19a, 19b) et la contre-interface (24, 25, 26, 27) est réalisée sous la forme d'au moins un évidement d'encliquetage (24, 25, 26, 27),
un élément d'adaptation (12) est disposé au niveau d'au moins un récipient stérile (1) de la pluralité de récipients stériles (1), est en particulier disposé de manière amovible, lequel réalise la contre-interface (24, 25) ou est réalisé par le biais de la contre-interface (24, 25), et
respectivement une pluralité de contre-interfaces (24, 25, 26, 27) sont réalisées au niveau d'au moins un récipient stérile (1) et/ou par le biais d'au moins un tamis (2), lesquelles contre-interfaces permettent de maintenir des plaques de marquage (13, 14) dans des positions différentes.

2. Système de marquage selon la revendication 1, **caractérisé en ce que** les plaques de marquage (13, 14) sont en plastique.

3. Système de marquage selon la revendication 1 ou 2, **caractérisé en ce que** la pluralité de plaques de marquage (13, 14) présentent des groupes de plaques de marquage (13, 14) de couleurs respectivement différentes.

4. Système de marquage selon l'une quelconque des revendications précédentes, **caractérisé en ce que** l'interface (19a, 19b) et la contre-interface (24, 25, 26, 27) comprennent des structures d'encliquetage complémentaires pouvant être mises en prise les unes avec les autres, en particulier pouvant être mises en prise les unes avec les autres de manière amovible.

5. Système de marquage selon l'une quelconque des revendications précédentes, **caractérisé en ce que** deux languettes d'encliquetage (19a, 19b) en forme de crochet espacées l'une de l'autre sont réalisées en tant qu'interface (19a, 19b) au niveau d'au moins une plaque de marquage (13, 14), lesquelles languettes présentent respectivement une base de languette d'encliquetage (20a, 20b) disposée au niveau de la plaque de marquage (13, 14) et un crochet d'encliquetage (21a, 21b) s'étendant de cette base d'encliquetage au côté opposé à la plaque de marquage (13, 14), dans lequel les crochets d'encliquetage (21a, 21b) sont tournés l'un vers l'autre ou détournés l'un de l 'autre et une section (22) déformable élastiquement est réalisée entre les languettes d'encliquetage (19a, 19b).

6. Système de marquage selon l'une quelconque des revendications précédentes, **caractérisé en ce que** la plaque de marquage (13, 14) présente une section de préhension (23a, 23b) qui est disposée en particulier côté bord sur la face, opposée à la section déformable (22), de la languette d'encliquetage (19a, 19b).

7. Système de marquage selon l'une quelconque des revendications précédentes, **caractérisé en ce que** la contre-interface (26, 27) d'au moins un tamis (2) est réalisée sous la forme d'au moins un évidement d'encliquetage (26, 27) pouvant être inséré dans une paroi de tamis.

8. Système de marquage selon l'une quelconque des revendications précédentes, comprenant en outre au moins un équipement de transport et/ou au moins un équipement de stockage et/ou au moins un support de nettoyage et de désinfection respectivement avec une contre-interface (24, 25, 26, 27) complémentaire à l'interface (19a, 19b).
